# EUROPEAN PATENT SPECIFICATION

(11) **EP 1 942 891 B1**
(45) Date of publication and mention of the grant of the patent: **23.03.2011**
(21) Application number: 06798441.9
(22) Date of filing: 22.09.2006
(51) Int. Cl.: A61K 31/445, A61K 31/135, A61K 31/165, A61K 31/381, A61K 31/00, A61K 31/55, A61P 25/24

(54) **NOVEL COMBINATION OF DRUGS AS ANTIDEPRESSANT**
NEUE ARZNEIMITTELKOMBINATION ALS ANTIDEPRESSIVA
NOUVELLE COMBINAISON DE MEDICAMENTS UTILISES COMME ANTIDEPRESSEUR

(30) Priority: 22.09.2005 JP 2005276222
(43) Date of publication of application: 16.07.2008
(73) Proprietor: Eisai R&D Management Co., Ltd., Tokyo 112-8088 (JP)
(72) Inventor: SAKAI, Kazuo, Tokyo 112-0002 (JP)
(74) Representative: HOFFMANN EITLE
(86) International application number: PCT/JP2006/319393
(87) International publication number: WO 2007/034990

(56) References cited:
- WO-A-2005/079784
- WO-A2-2005/051297
- ANONYMOUS: "Treatment-resistant depression" WIKIPEDIA, [Online] Retrieved from the Internet: URL:http://en.wikipedia.org/wiki/Treatment -resistant_depression> [retrieved on 2009-06-16]

## Description

### Technical Field

The invention relates to a novel combination of compounds that act on the central nervous system and that can be used as an antidepressant. The invention also relates to using the anti depressant combination or composition, in a method for the treatment of depression.

### Background Art

Wood disorders include a group of many diseases. "Depression (or depressive state)" is classified as a mood disorder and is the most common mental disease. It tends to increase year by year due to changes in social environments and is becoming a big social problem not only in Japan but also in foreign countries. No uniform and definite therapeutic method has been established for depression because there are ambiguities in the mechanisms of action that cause depression and the causes for the onset of depression.

Among the currently available therapeutic methods, drug therapy is believed to be the most effective and, because of the possibility for uniform efficacy and for improvement within a short period of time, many studies have been carried out for the development of antidepressants. With regard to the mechanism of action for depression, the concentration of monoamine neurotransmitters, such as serotonin and noradrenaline(norepinephrine), decrease in the synaptic cleft which is the place for neurotransmission between neurons in the brain. Coppen, British J. Psychiatry, 113:1237-1264 (1967). The monoamine neurotransmitters are released from the presynaptic axon terminal of the neurons into the synaptic cleft and specifically bind to a receptor existing in the postsynaptic dendrites of another neuron whereby they contribute to signal transmittance between neurons. The released monoamine neurotransmitters are subjected to reuptake from the synaptic cleft by a transporter existing in the presynaptic axon terminal and a part of the neurotransmitters are decomposed by monoamine oxidase while another part of the neurotransmitters are subjected to uptake into synaptic vesicles and are again released to the synaptic cleft.

The first antidepressants were tricyclic and tetracyclic and were used based on the finding that the concentration of monoamine neurotransmitters, such as serotonin and noradrenaline (norepinephrine), in the synaptic cleft decreases in depression. The tricyclic and tetracyclic antidepressants bind to a transporter of neurotransmitters existing in the presynaptic axon terminals to inhibit the reuptake action of the neurotransmitters in the transporter, whereupon they increase the concentration of the neurotransmitters released into the synaptic cleft. However, they bind not only to transporters for neurotransmitters such as serotonin and noradrenaline but also to other receptors, e.g., muscarine-like acetylcholine receptors, histamine H₁ receptors, α₁α₂ adrenaline receptors, existing in postsynaptic dendrites, which causes various side effects that are induced by the antagonistic actions to the other receptors.

In order to avoid the side effects of tricyclic and tetracyclic compounds, monoamine oxidase inhibitors (MAOIs) were developed. Monoamine oxidase inhibitors irreversibly inhibit the monoamine oxidase which decomposes and metabolizes information transmitters which are subjected to reuptake into presynaptic axon terminals, and pools the information transmitter to the presynaptic axon terminals without metabolizing it, resulting in an increase in monoamine concentration of the synaptic cleft. Unlike tricyclic and tetracyclic antidepressants, monoamine oxidase inhibitors do not bind to muscarine-like acetylcholine receptors, histamine H₁ receptors and α₁·α₂ adrenaline receptors. However, until new monoamine oxidase is biosynthesized, monoamine is not metabolized but is pooled in the presynaptic axon terminals. When a patient ingests food containing tyramine, which excessively induces monoamine (e.g., particularly noradrenalin), severe side effects such as sudden hypertension may occur.

Substances which have been developed for reducing the side effects caused by tricyclic and tetracyclic antidepressants and by monoamine oxidase inhibitors are selective serotonin reuptake inhibitors (SSRI) and selective serotonin-noradrenalin reuptake inhibitors (SNRI) which selectively inhibit the reuptake of serotonin and noradrenalin. As their names show, reuptake of serotonin and noradrenalin of synaptic cleft by presynaptic axon terminals is inhibited in a serotonin- and/or noradrenalin-selective manner whereby they do not cause side effects such as antagonistic actions to other receptors or an excessive liberation of monoamine

SSRIs have been recognized throughout the world as antidepressants which are most effective and have little-side effects. However, compounds classified as SSRIs have little similarity and/or common features in view of their chemical structures. In addition, there are many cases where even if one compound classified as an SSRI has efficacy, another SSRI may have no efficacy. . Accordingly, there is an increasing need for drugs which can efficiently treat depression in a continued and predicted manner.

There are many cases where SNRIs, such as milnacipran and duloxetine, do not show efficacy. Accordingly, there is an increasing need for drugs which can treat depression in a continued and predicted manner.

"Depression" has been believed to be an "emotional disorder" where loss of joy and loss of volition are the most pronounced characteristics and it has been also believed that decrease in activity, decrease in processing ability, physical disorders, and the like, which are accompanied with depression are induced as a result of the emotional disorder. In fact, in drug therapy for depression, emphasis, is put on a selective increase or decrease of concentration of serotonin and/or noradrenalin of the synaptic cleft which is thought to be closely related to emotion and there have been attempts for providing drugs having better antidepressive actions than before by the combination of two or more antidepressants acting on serotonin and/or noradrenalin.

Cholinesterase inhibitors, such as donepezil, rivastigmine, tacrine, galanthamine, metrifonate, neostigmine and physbstigmine, have not been used in the treatment of depression so far.

In WO 2005/051297, methods utilizing a combination of one or more cholinesterase inhibitors and one or more antidepressants for achieving a desirable weight loss in an overweight or obese individual are described. Said methods are specified as also being applicable in obese or overweight individuals further being diagnosed with and suffering from depression.

In WO 2005/079784, the use of a cholinesterase inhibitor in augmenting the antidepressant therapy of vascular depression by e.g. SSRIs and SNRIs is disclosed. Vascular depression results from vascular damage to fronto-subcortical structures. It therefore represents an affective disorder caused by physical diseases including brain organic diseases such as a cerebrovascular disorder and brain tumor and therefore has to be differentiated from depression as discussed herein before.

### Disclosure of the Invention

It is an object of the invention to provide a novel-composition or combination for use in the treatment of depression, particularly major depression. Another object of the invention is to provide the use of a new combination of drugs in a method for treating depression, particularly major depression.

The inventors have plaid attention to their idea that a cholinesterase inhibitor used as a therapeutic agent for Alzheimer's disease may achieve an effective antidotal action for depression. As a result, it has been unexpectedly discovered that when a cholinesterase inhibitor is combined with a selective serotonin reuptake inhibitor, or milnacipran, it is possible to treat depression, in particular major depression, which is not effectively treated solely with a selective serotonin reuptake inhibitor, or milnacipran , respectively.

In a first aspect, the invention provides a pharmaceutical composition for use in the treatment of depression (e.g., major depression) comprising a cholinesterase inhibitor in combination with a selective serotonin reuptake inhibitor (such a compound may be an enantiomer, a diastereomer, a tautomer, a pharmaceutically acceptable salt, an active metabolite, a prodrug thereof or a solvate thereof).

In another aspect, the invention provides use of the aforementioned pharmaceutical composition for the manufacture of a medicament for the treatment of major depression.

In a second aspect, the invention provides a pharmaceutical composition for use in the treatment of depression (e.g., major depression) comprising a cholinesterase inhibitor in combination with milnacipran (such a compound may be an enantiomer, a diastereomer, a tautomers, a pharmaceutically acceptable salt, an active metabolite, a prodrug thereof or a solvate thereof).

In another aspect, the invention provides uses of the aforementioned pharmaceutical composition for the manufacture of a medicament for the treatment of depression (e.g., major depression).

In a third aspect, the invention provides (a) pharmaceutical compositions comprising, (b) pharmaceutical combinations comprising, and (c) kits comprising, (A) a cholinesterase inhibitor, and (B) (i) a selective serotonin reuptake inhibitor, or (ii) milnacipran, an enantiomer thereof, a diastereomer thereof, a pharmaceutically acceptable salt thereof, an enantiomer of a pharmaceutically acceptable salt thereof, or a diastereomer of a pharmaceutically acceptable salt thereof; The selective serotonin reuptake inhibitor is (a) fluoxetine, an enantiomer thereof, a pharmaceutically acceptable salt thereof, or an enantiomer of a pharmaceutically acceptable salt thereof; (b) fluvoxamine or a pharmaceutically acceptable salt thereof; (c) paroxetine, an enantiomer thereof, a diastereomer thereof, a pharmaceutically acceptable salt thereof, an enantiomer of a pharmaceutically acceptable salt thereof, or a diastereomer of a pharmaceutically acceptable salt thereof; (d) sertraline, an enantiomer thereof, a diastereomer thereof, a pharmaceutically acceptable salt thereof, an enantiomer of a pharmaceutically acceptable salt thereof, or a diastereomer of a pharmaceutically acceptable salt thereof; or (e) escitalopram, or a pharmaceutically acceptable salt thereof.

In a fourth aspect, the invention provides (a) pharmaceutical compositions comprising, (b) pharmaceutical combinations comprising, and (c) kits comprising, (A) donepezil or a pharmaceutically acceptable salt thereof and (i) a selective serotonin reuptake inhibitor; or (ii) milnacipran or a pharmaceutically acceptable salt thereof. The selective serotonin reuptake inhibitor is (a) fluoxetine or.a pharmaceutically acceptable salt thereof; (b) fluvoxamine or a pharmaceutically acceptable salt thereof; (c) paroxetine or a pharmaceutically acceptable salt thereof; (d) sertraline or a pharmaceutically acceptable salt thereof, or (e) escitalopram, or a pharmaceutically acceptable salt thereof

The meritorious effects of the invention are summarized as follows. Antidepressants usually provide beneficial effects only after two weeks to two months after the initial administration to the patient. It was surprisingly discovered that the pharmaceutical combination of the invention provided beneficial effects within about one week after the initial administration to the patient. It has also been noted that the effect is achieved, for example, when administration of a cholinesterase inhibitor is started again when symptoms worsen as a result of discontinuation or decrement of administration of the cholinesterase inhibitor.

### Best Mode for Carrying Out the Invention

The embodiments mentioned hereinafter are examples for illustrating the invention and they are not intended to limit the invention. All technical terms, scientific terms and professional terms used throughout the specification have the same meanings which are generally understood by persons skilled in the technical field to which the invention belongs and they are not intended to be used for the illustration of only a specific embodiment and are not intended to be limited thereto. Any method and material being similar to or identical with those which are used in the specification are able to be used in conducting or testing the invention and, with regard to the preferred methods and materials, the following description may be referred to. The invention may be carried out in various embodiments so far as they are not out of the gist of the invention.

All prior art documents and patent documents including laid-open patents and published patents cited in the specification are incorporated in the specification as references and can be used for carrying out the invention.

"Depression" which is classified as "296. xx: major depressive disorders" and "minor depressive disorders" belonging to "Mood Disorders" according to the diagnostic standard of DSM-IV-TR (published in 2000) which is a revised edition of DSM-IV or "American Psychiatric Association, Diagnostic and Statistical Manual of Mental Disorders; Fourth Edition." The major depressive disorders are further classified as "single episode" which is a major depressive episode for the first time (single) and "recurrent episode" which is experiences of two or more major depressive episodes in the past.

According to a diagnostic standard of ICD-10 or "International Statistical Classification of Disease and Related Health Problems; Tenth Edition" of the World Health Organization (WHO), "depression" is classified as "F 32: Depressive episode" and "F 33: Recurrent depressive disorder" in "Chapter V (F): Mental and Behavioral Disorders."

The terms "mixed" and "bipolar" used in those diagnostic standards include an episode of "depression (or depressive state)" and also include an episode of "mania (or manic state)."

"Depression (or depressive state)" which is an object of the present invention will be further illustrated based on various diagnostic standards.

According to DSM-IV-TR, a "major depressive episode" is satisfied by the following criteria A-E.
A. Five (or more) of the following symptoms are present within the same two weeks and changes from the function before the disease are noted: (1) depressive mood almost throughout the day or almost everyday shown either by clear expression by himself/herself (such as sad feeling or empty feeling) or by observation by others (such as he/she is weeping) (in the case of children and young people, irritated mood may be available as well); (2) significant decrease in interest or joy in all of or nearly all of activities almost throughout the day or almost everyday (shown either by his/her expression or by observation by others); (3) significant decrease in body weight or increase in body weight (such as changes of not less than 5% of body weight per months) where no diets are followed or decrease or increase in appetite almost everyday (in the case of small children, the fact that expected increase in body weight is not noted is to be also taken into consideration); (4) Insomnia or hypersomnia in almost everyday; (5) psychomotor impatience or restraint in almost everyday (which is able to be observed by others and is not a subjective sense such as merely restless or slow); (6) easy fatigability or decrease in vitality in almost every day; (7) sense of valuelessness or excessive or inappropriate angst in almost every day (sometimes just delusive; not the sense of sin just to feel guilty oneself or to become the disease); (8) decrease in thinking power or in power of concentration or difficulty in making a decision is noted in almost everyday (either by his/her own statement or by observation by others); (9) repetitive thinking of death (not only worrying about death), repetitive desire for suicide without a special plan or an apparent plan for suicide intention or for committing suicide. At least one of the symptoms is (1) depressive mood or (2) loss of interest or joy. (Note: Symptoms clearly caused by general physical diseases or by delusion or hallucination being not corresponding to mood are excluded.)
B. The symptom does not satisfy the standard for a mixed episode
C. The symptom causes a clinically significant pain or a function disorder in society, profession or other important fields.
D. The symptom is not due to a direct physiological action of a substance (e.g., abused drug, administration of drug) or due to general physical diseases (e.g., hypothyroidism).
E. The symptom is unable to be well explained by bereavement reaction. Thus, the symptom is characterized by continuance for more than two months after his/her beloved person passed away or by the presence of significant functional insufficiency, abnormal adhesion to the sense of valueless idea, desire for suicide, psychopathic symptom or mental motion suppression.

When the above-mentioned diagnostic standards are satisfied, the symptom will be then able to be diagnosed as either "296.2x: major depressive disorder - single episode" or "296.3x: major depressive disorder - recurrent episode."

When the following three standards of A to C are satisfied, it is diagnosed as "296.2x: major depressive disorder - single episode."
A Presence of single episode of major depressive disorder;
B. It is unable to be well explained by major depressive episode or ataxic motion disorder and does not overlap with schizophrenia, schizophrenic disorder, delusional disorder or unspecified psychopathic disorder; and
C. Manic episodes, mixed episodes and light manic episodes are not present (When all of manic, mixed and light manic episodes are induced by a substance or a therapy or when they are due to a direct physiological action of general physical diseases, such an exception is not applied.)

When a case does not satisfy the diagnostic standard for "296.2x: major depressive disorder - single episode" but satisfied all of the following standards A to C, such a case is diagnosed as "296.3x: major depressive disorder - recurrent".
A. Presence of two or more major depressive episodes (note: in order to judge that they are independent episodes, a period which does not satisfy the standard for major depressive episode should be present at least for continued two months);
B. It is unable to be well explained by major depressive episode or ataxic motion disorder and does not overlap with schizophrenia, schizophrenic disorder, delusional disorder or unspecified psychopathic disorder; and
C. Manic episodes, mixed episodes and light manic episodes are not present. 2. ICD-10

In accordance with ICD-10, "depression" is classified and diagnosed as follows.

F 32: Depressive episode. In the three types of typical depressive episodes (light (F 32.30), medium (F 32.1) or severe (F 32.2 and F 32.3), patients are usually bothered by depressive mood and increase in easy fatigability or decrease in activity due to loss of interest and joy and decrease in vitality. Even after a little endeavor, much fatigue is usually noted. Other generic symptoms are as follows: (a) decrease in concentrating power and power of attention; (b) lowering in self evaluation and self confidence; (c) guilty sense and sense of no value (they are also noted even in the case of light episode); (d) hopeless and pessimistic way of thinking for the future; (e) sense or act for self injury or suicide; (f) insomnia; and (g) anorexia

Mental depression shows little change day by day and it is often non-reactive to environments although, when days lapse, a specific change within a day may take place. As in the case of manic episodes, there is a clear difference among individuals in its clinical image and, particularly in adolescence, non-typical symptoms are usually noted. In some cases, much anxiety, distress and mental motility are sometimes superior to depressive symptom and, in addition, changes in mood may become latent when character of being apt to be stimulated, excessive taking of alcoholic drinks, performance act, worsening of already-existing acrophobia and compulsive symptom or symptoms such as hypochondriac insistence are added. For the diagnosis of depressive episode, continuance for at least two weeks is usually necessary independently of degree of severeness and, if very severe and sudden onset is noted, it is acceptable to diagnose within a shorter period.

There are some cases where some of the above-mentioned symptoms are significant or where characteristic symptom which has been widely noted to have a particularly clinical significance is noted. Some examples of the most typical cases of "physical" symptoms as such are as follows. Thus, joy and interest are lost for the activity which is usually thought to be pleasant; emotional reaction is lacking to the situation or matter which usually enjoyable; awaking in the morning is two hours or more earlier than usual; depressive feeling is significant during the morning; apparent psychomotor impatience or restraint is objectively noted (being noticed or reported by others); clear anorexia; body weight reduction (it is often defined as 5% or more during the past one month); and clear reduction in sex drive. When about four of the above-mentioned symptoms are clearly noted, it is usually recognized to be "physical syndrome is available". However, even when physical syndrome is noted only in two or three items, that may be also included within such a category provided that the degree is very severe.

Categories for mild depressive episode (F 32.0), moderate depressive episode (F 32.1) and severe depressive episode (F 32.2 and F 32.3) which are illustrated in detail hereinafter should be used only for the single (first) depressive episode. Other depressive episodes should be classified as one of subtypes of recurrent depressive disorders (F 33).

F 32.0: Mild depressive episode. Diagnostic guideline: Depressive mood, loss of interest and joy and easy fatigability are usually considered to be the most typical symptoms for depression. When at least two of them and further when two or more other symptoms mentioned in the following F 32 are noted, diagnosis is established. Any of such symptoms recognized as such should not be significant and the shortest continuing time for the episode as a whole is about two weeks. Patients suffering from mild depressive episode usually have some difficulties in continuing daily work and social activity due to the symptom but are not completely non-functioned.

F 32.1: Moderate depressive episode. Diagnostic guideline: When two or more of the three most typical symptoms listed in a mild depressive episode (F 32.0) are noted and further when three or more (four is preferred) of other symptoms are noted, diagnosis is established. Although several symptoms tend to become to a significant extent, that is not a necessary matter if broad symptoms are present as a whole. The shortest continuing time of the episode as a whole is about two weeks. Patients suffering from moderate depressive episode are usually able to become considerably difficult for continuing social, professional, or domestic activity.

F 32.2: Severe depressive episode without psychotic symptoms. In severe depressive episode, patients usually show considerable agony and agitation provided that the control is not significant. They are apt to have loss of the spirit of self-respect, sense of no value and guilty feeling and, particularly in severe cases, risk of suicide is very high. In severe depressive episode, it is presumed that physical symptoms are present nearly at all times.

Diagnostic guideline: In addition to all of typical three symptoms mentioned for mild and moderate depressive episode (F 32.0 and F 32.1), four or more of other symptoms are noted and some of them should be severe. However, if important symptoms such as agitation and mental motility control are significant, there are some cases that patients do not want to or are unable to explain many symptoms in detail. Even in such a case, it will be appropriate to judge to be severe episode as a whole. Usually the depressive episode should continue for two weeks or longer but, if the symptom is very severe and in a sudden onset, such a diagnosis is acceptable even in the case of shorter than two weeks.

During the period of severe depressive episode, it is almost impossible for a patient, except a very limited case, to continue social, professional or domestic activity. This category should be used only for a single severe depressive episode without psychopathic symptoms. For an episode thereafter, a lower classification of recurrent depressive disorder (F 33) should be used.

F 32.3: Severe depressive episode with psychotic symptoms. Diagnostic guideline: This is a severe depressive episode which satisfies the diagnostic standard for severe depressive episode without psychotic symptoms (F 32.2) and accompanied by delusion, hallucination or depressive stupor. Delusion is usually that relating to guilt, poverty, imminent accident, guilty conscience, etc. Auditory hallucination and olfactory hallucination are usually slander, chorus of criticism, putrefied dirt, putrefied smell of meat, etc. Severe psychiatric movement control may results in stupor.

Differential diagnosis: Depressive stupor should be sifted from catatonic schizophrenia (F 20.2), dissociated stupor (F 44.2) and organic stupor. This category should be used only for a single episode of severe depression with psychotic symptoms. For an episode thereafter, a lower classification for recurrent depressive disorder (F 33) should be used.

F 33: Recurrent depressive disorder. This disorder is characterized in that "depressive" episode specified as any of mild (F 32.0), moderate (F 32.1) and severe (F 32.2 and F 32.3) episodes is repeated and that independent episode history of hyperthymia and overactivity satisfying the diagnostic standard of mania (F 30.1 and 30.2) is lacking. This category should be still used even when there is the evidence that hyperthymia and overactivity for a short period to an extent of satisfying the diagnostic standard for mild mania (F 30.0) occur immediately after depressive episode (in some cases, being clearly induced by therapy of depression). Each of age for onset, degree of severeness, duration and frequency of episode of depression is various. Usually, the initial episode appears later as compared with the case of bipolar disorder and an average age for onset is forties. Each episode endures for three months to twelve months (the median for the duration is about six months). Between each episode, complete recovery is usually noted but, in some patients, depression may longer. Even in such a case, this category should be used. Regardless of the degree of severeness, each episode is often induced by accidents during a stressful life. In many civilized areas, both of each episode and each long-lasting depression are noted in twice as much in males than in females.

Risk of finding a manic episode in patients suffering from recurrent depressive disorder does not completely disappear even if depressive episode is experienced for several times. Once a manic episode is noted, diagnosis should be changed to bipolar disorder.

Recurrent depressive disorder is sub-classified as follows by such a manner that, firstly, type of the episode at present is specified and then (provided that full information is able to be achieved) a type which is dominant in all episodes is specified.

F 33.0; Recurrent depressive disorder; current episode mild. Diagnostic guideline: For a definite diagnosis: (a) diagnostic standard for recurrent depressive disorder (F 33) should be satisfied and the episode at present should satisfy the diagnostic standard for mild depressive episode (F 32.0); and (b) at least episodes for two times continue for two weeks at the shortest and they should be separated by several months where no apparent mood disorder is noted.

F 33.00: without physical syndrome (refer to F 32.00)

F 33.01: with physical syndrome (refer to F 32.01)

F 33.1 Recurrent depressive disorder, current episode moderate

For a definite diagnosis: (a) diagnostic standard for recurrent depressive disorder (F 33) should be satisfied and the episode at present should satisfy the diagnostic standard for moderate depressive episode (F 32.1); and (b) at least episodes for two times continue for two weeks at the shortest and they should be separated by several months where no apparent mood disorder is noted.

F 33.10: without physical syndrome (refer to F 32.10)

F 33.11: with physical syndrome (refer to F 32.11)

F 33.2: Recurrent depressive episode; current episode severe without psychotic symptoms. For a definite diagnosis: (a) diagnostic standard for recurrent depressive disorder (F 33) should be satisfied and the episode at present should satisfy the diagnostic standard for severe depressive episode without psychotic symptoms (F 33.2); and (b) at least episodes for two times continue for two weeks at the shortest and they should be separated by several months where no apparent mood disorder is noted.

If not, other recurrent mood disorder (F 38.1) should be used as diagnosis. If necessary, a type which is dominant in the previous episode (mild or moderate, severe or unknown) may be specified.

F 33.3: Recurrent depressive disorder; current episode severe with psychotic symptoms. In order to issue a definite diagnosis for this category, all of the following standards should be satisfied: (a) diagnostic standard for recurrent depressive disorder (F 33) should be satisfied and the episode at present should satisfy the diagnostic standard for severe depressive episode with psychotic symptoms (F 33.3); and (b) at least episodes for two times continue for two weeks at the shortest and they should be separated by several months where no apparent mood disorder is noted.

F 33.4: Recurrent depressive disorder; currently in remission In order to issue a definite diagnosis for this category, all of the following standards should be satisfied: (a) in the past, diagnostic standard for recurrent depressive disorder (F 33) had to be satisfied and, at present, diagnostic standard for any severe depressive episode or any other disorder under F 30 to F 39 should not be satisfied; and (b) at least episodes for two times continue for two weeks at the shortest and they should be separated by several months where no apparent mood disorder is noted.

Incidentally, this category (F 33.4) may be also used even in the case where a patient is treated for prevention of recurrence.

F 33.8: Other recurrent depressive disorders

F 33.9: Recurrent depressive disorder; unspecified

The expression reading "treating" or "able to be treated" used in the invention means a partial or whole remission for a morbid state where no or little therapeutic effect is noted by known drugs (preferably, selective serotonin reuptake inhibitors, milnacipran or duloxetine) or, for a morbid state where onset is noted again upon termination of administration of the drug even when remission is once noted partially or wholly by drug therapy (preferably, selective serotonin reuptake inhibitors, milnacipran or duloxetine), it means the case where no remission happens even when administration of the drug is terminated after a partial or whole remission and, even if remission happens, the period which does not satisfy the standard for mood disorder is significantly prolonged as compared with the previous repetition period.

For evaluation of the state of depression, rating scales which has been widely used are, for example, Hamilton's rating scale for depression (HAM-D) (Hamilton, M., Journal of Neurology, Neurosurgery & Psychiatry, 23: 56-62 (1960) and Hamilton, M., "Development of rating scale for primary depressive illness", British Journal of Social and Clinical Psychology, 6: 278-296 (1967)), Montgomery-Asberg's rating scale for depression (MADRS) (Montgomery, SA, Asberg, M, "A new depression scale designed to be sensitive to change", British Journal of Psychiatry, 134: 382-389 (1979)) and Clinical Global Impression (CGI) Rating Scale for Depression (Guy, W, "ECDEU Assessment Manual for Psychopharmacology", revised edition, U. S. Department of Health, Education and Welfare, Bethesda, MD. (1976)).

When the invention is illustrated using Hamilton's rating scale for depression (HAM-D) as an example, the case is diagnosed as severe where the point is 20 or higher and, as the point decreases, the symptom is diagnosed as being improved.

"Patient" means an animal and, preferably, a mammal "Mammal" includes all kinds of animals classified as human and non-human mammals (such as mice, rats, hamsters, guinea pigs, rabbits, pigs, dogs, cats, horses, cattle and monkeys). Preferably, the mammal in the present specification is human. In that case, the term "patient" includes adults and children and also includes males and females. "Children" includes babies and adolescents.

"Treatment (or therapy)" usually refers to acquisition of a desired pharmacological effect and/or physiologic effect. These effects are prophylactic in terms of completely or partially preventing a disease and/or a symptom, and therapeutic in terms of partially or completely curing a disease and/or an adverse effect caused by a disease. As used herein, "treatment (or therapy)" includes any treatment of a disease of a patient, particularly human, including, for example, at least one of the following treatments (a) to (c): (a) to prevent a disease or a symptom in a patient who is suspected of being predisposed to the disease or the symptom but not yet diagnosed to be so; (b) to inhibit a symptom of a disease, that is, to inhibit or delay the progress of the symptom, (c) to alleviate a symptom of a disease, that is, to reverse or eliminate the symptom of the disease, or to reverse the progress of the symptom.

"Prodrug" means a product where "active ingredient of drug" (which means a "drug" opposite to a prodrug) is chemically modified to an inactive substance with an object of improvement of bioavailabilrty, mitigation of side effect, etc. and means a drug which is metabolized to an active ingredient *in vivo* after being absorbed and achieves an action. Accordingly, the term "prodrug" stands for any compound which has a low intrinsic activity as compared with the corresponding "drug" but, upon administration to a biological system, produces the "drug" substance as a result of a spontaneous chemical reaction, an enzymatically catalytic reaction or a metabolic reaction. Examples of a prodrug are compounds where amino group, hydroxyl group, carboxyl group, etc. of the drug are acylated, alkylated, phosphorylated, borated, carbonylated, esterified, amidated or urethanized and a prodrug means a derivative which has a chemically or metabolically degradable group and shows a pharmaceutical activity by hydrolysis or solvolysis or by decomposition under a physiological condition. However, the above-exemplified groups are not comprehensive but merely typical ones and persons skilled in the art are able to prepare other known various kinds of prodrugs by a known method from cholinesterase inhibitors, selective serotonin reuptake inhibitors, milnacipran or duloxetine.

"Active metabolite" is a substance which shows a phenomenon where an action is enhanced or achieved by a drug-metabolizing enzyme.

Known cholinesterase inhibitors can be used in the invention as an effective ingredient. Examples thereof are acetylcholinesterase inhibitors and butyrylcholinesterase inhibitors, wherein the former is preferred. Specific examples of the cholinesterase inhibitor are as follows, although they are non-limitative: donepezil (1-benzyl-4-[(5,6-dimethoxy-1-indanon)- 2-yl]methylpiperidine); rivastigmine (3-[(S)-1-(dimethylamino)ethyl]phenyl N-ethyl-N-methylcarbamate); tacrine (1,2,3,4-tetrahydro-9-acridineamine); galanthamine (4aS,6R,8aS)-4a,5,9,10,11,12-hexahydro-3-methoxy-11-ethyl-6H-benzo-furo(3a,3,2-ef)-[2]-benzoazepin-6-ol); metrifonate (dimethyl(2,2,2-trichloro-1-hydroxyethyl) phosphonate); neostigmine (3-(dimethylcarbamoxyphenyl)-trimethyl-amonium); and physostigmine: (3aS-cis)-1,2,3,3a,8,8a-hexahydro-1,3a,8- trimethylpyrrolo[2,3-b]indol-5-ol-methylcarbamate (ester). Particularly preferred are donepezil, rivastigmine and galanthamine, and the most preferred is donepezil. In addition, with respect to the cholinesterase inhibitors used in the invention as an effective ingredient, it is also possible to use an enantiomer thereof, a pharmaceutically acceptable salt of an enantiomers thereof, a diastereomer thereof, a pharmaceutically acceptable salt of a diastereomers thereof, a tautomer thereof, a pharmaceutically acceptable salt of a tautomer thereof, a pharmaceutically acceptable salt thereof, an active metabolite thereof, a prodrug thereof, and a solvate thereof. The cholinesterase inhibitor of the invention includes such isomers as all geometric isomers due to its chemical structure, optical isomers based on its asymmetric carbon, rotational isomers, stereoisomers, tautomers and mixtures thereof. Any descriptions for convenience sake do not limit the compound, which may be any one of isomers or a mixture thereof. Therefore, in case of the cholinesterase inhibitor of the invention is a stereoisomer having asymmetric carbon in the molecule, or a racemate thereof, both are included in the compound without any limitations. In addition, any polymorphic crystals, a single or a mixture thereof may be included in the invention without limitation.

The cholinesterase inhibitor used as an effective ingredient in the present invention may be produced by a known method. Donepezil can be produced by a process disclosed, for example, in Japanese Patent Laid-Open No. 01/079,151, Japanese Patent Nos. 2,578,475, 2,733,203 and 3,078,244 or U. S. Patent No. 4,895,841. Donepezil hydrochloride is also available as a pharmaceutical preparation. Galanthamine can be produced by a process disclosed, for example, in U. S. Patent No. 4,663,318, WO 88/08708, WO 97/03987 and U. S. Patent Nos. 6,316,439, 6,323,195 and 6,323,196. Tacrine can be produced by a process disclosed, for example, in U. S. Patent Nos. 4,631;286, 4,695,573 and 4,754,050, -WO 88/02256, U. S. Patent Nos. 4,835,275, 4,839,364 and 4,999,430 and WO 97/21681. Rivastigmine can be produced by a process disclosed, for example, in European Patent No. 193,926, WO 98/26775 and WO 98/27055. Metrifonate, neostigmine, physostigmine, etc. may be also produced by known processes mentioned in prior art documents.

With regard to the selective serotonin reuptake inhibitor used as an effective ingredient in the invention, those mentioned, for example, in Japanese Patent Laid-Open No. 2002/542,287 may be used. Preferred examples thereof are as follows, although they are non-limitative: fluvoxamine (5-methoxy-1-[4-(trifluoromethyl)phenyl]- 1-pentanone(E)-O-(2-aminoethyl) oxime); fluoxetine (N-methyl-3-(p-trifluoromethylphenoxy)-3-phenylpropyl-amine); paroxetine (-)-(3S,4R)-4-(4-fluorophenyl)-3-(3,4-methylenedioxy)phenoxymethyl)piperidine; sertraline (+)-(1S-cis)-4-(3,4-dichlorophenyl)-1,2,3,4- tetrahydro-N-methyl-1-naphthylamine); and escitalopram (+)-(S)-1-[3-(dimethylamino)propyl]-1-(4-fluorophenyl)-1,3-dihydrobenzofuran-5-carbonitrile. Particularly preferred are fluvoxamine, fluoxetine, paroxetine and sertraline. In addition, with respect to the selective serotonin reuptake inhibitor used in the invention as an effective ingredient, it is also possible to use an Enantiomer thereof, a pharmaceutically acceptable salt of an enantiomer thereof, a pharmaceutically acceptable salt of a diastereomers thereof, a diastereomer thereof, a tautomer thereof, a pharmaceutically acceptable salt of a tautomer thereof, a geometrical isomer such as Z- or E-type thereof, a pharmaceutically acceptable salt thereof, an active metabolite thereof, a prodrug thereof, and a solvate thereof. The selective serotonin reuptake inhibitor of the invention includes such isomers as all geometric isomers due to its chemical structure, optical isomers based on its asymmetric carbon, rotational isomers, stereoisomers, tautomers and mixtures tereof. Any descriptions for convenience sake do not limit the compound, which may be any one of isomers or a mixture thereof, Therefore, in case of the selective serotonin reuptake inhibitor of the invention is a stereoisomer having asymmetric carbon in the molecule, or a racemate thereof, both are included in the compound without any imitations In addition, any polymorphic crystals, a single or a mixture thereof may be included in the present invention without limitation.

The selective serotonin reuptake inhibitor used as an effective ingredient in the present invention may be produced by a known method. Fluvoxamine can be produced by a process disclosed, for example, in Japanese Patent Publication No. 60/026,776 and U. S. Patent No. 4,085,225. Fluoxetine can be produced by a process disclosed, for example, in U. S. Patent No. 4,314,081, WO 98/33496 and Japanese Patent Publication No. 59/039,418. Paroxetine can be produced by a process disclosed, for example, in U. S. Patent Nos. 4,007,196 and 3,912,743 and Japanese Patent Publication Nos. 5,9/046,216 and 59/048,826. Sertraline can be produced by a process disclosed, for example, in U. S. Patent No. 4,536,518 and Japanese Patent Publication No. 60/005,584.

Milnacipran (1R,2S)-N,N-diethyl-2-aminomethyl-1-phenylcyclopropanecarboxamide) is able to be produced by a known process. Milnacipran can be produced by a process disclosed, for example, in European Patent No. 200,638, Japanese Patent Publication No. 05/067,136, French Patent No. 2,581,060 and U. S. Patent No. 4,478,836.

Milnacipran of the invention includes such isomers as all geometric isomers due to its chemical structure, optical isomers based on its asymmetric carbon, rotational isomers, stereoisomers, tautomers and mixtures thereof. Any descriptions for convenience sake do not limit the compound, which may be any one of isomers or a mixture thereof. Therefore, in case of the milnacipran of the present invention is a stereoisomer having asymmetric carbon in the molecule, or a racemate thereof, both are included in the compound without any limitations. In addition, any polymorphic crystals, a single or a mixture thereof may by included in the present invention without limitation.

The cholinesterase inhibitor, selective serotonin reuptake inhibitor, or milnacipran of the invention may be in a form of a pharmaceutically acceptable salt thereof. For example, it is an inorganic acid such as hydrochloric acid, hydrobromic acid, hydriodic acid, sulfuric acid and phosphoric acid and an organic acid such as p-toluenesulfonic acid, methanesulfonic acid, oxalic acid, p-bromophenylsulfonic acid, carbonic acid, succinic acid, citric acid, benzoic acid and acetic acid Examples of the pharmaceutically acceptable salt are hydrochloride, sulfate, pyrosulfate, bisulfate, sulfite, bisulfite, phosphate, monohydrogen phosphate, dihydrogen phosphate, metaphosphate, pyrophosphate, chloride, bromide, iodide, acetate, propionate, decanoate, caprylate, acrylate, formate, isobutyrate, caprate, heptanoate; propionate, oxalate, malonate, succinate, sebacate; fumarate, maleate, benzoate, hydroxybenzoate, methoxybenzoate, phthalate, terephthalate, sulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate and naphthalene-2-sulfonate although they are non-limitative. With regard to the acetylcholinesterase inhibitors of the invention, donepezil, rivastigmine and galanthamine are preferred to be in a form of hydrochloride, tartrate and hydrobromide, respectively. With regard to the selective serotonin reuptake inhibitor of the invention, fluoxetine, fluvoxamine, paroxetine and sertraline are preferred to be in a form of hydrochloride, maleate, hydrochloride and hydrochloride, respectively. Milnacipran of the invention is preferred to be in a form of hydrochloride and hydrochloride, respectively.

The cholinesterase inhibitor, selective serotonin reuptake inhibitor, milnacipran, a pharmaceutical salt thereof and the like of the invention may be an anhydride or a solvate, if it is present. The solvate may be either a hydrate or non-hydrate, but a hydrate is preferable. To make the non-hydrate, for example, alcohol (e.g.. methanol, ethanol and n-propanol) and dimethylformamide can be used.

Among the cholinesterase inhibitor, selective serotonin reuptake inhibitor, milnacipran, a pharmaceutical salt thereof and the like of the present invention, a commercially available compound can be easily available from chemical manufacturers, etc.

The cholinesterase inhibitor of the invention may be formulated in any combination with the selective serotonin reuptake inhibitor, or milnacipran. A combination of one or more cholinesterase inhibitor(s) with one or more selective serotonin reuptake inhibitor(s), or milnacipran is also acceptable.

The pharmaceutical composition used in the combination therapy of the invention is also provided as the pharmaceutical composition comprising a drug containing a cholinesterase inhibitor with a drug containing a selective serotonin reuptake inhibitor or milnacipran. In a combination therapy according to the invention, each of the combined ingredients in an effective dose thereof may be directly administered at the same time or each of them in an effective dose thereof may be administered with time intervals. It is also possible that each of the combined ingredients may be made into a pharmaceutical composition prepared by a commonly used method in an effective dose administered at the same time or administered with time intervals. It is further possible that, in the combination therapy according to the invention, the ingredients to be combined are just compounded by mixing and the resulting pharmaceutical preparation in an effective dose is administered, or they are made into respective pharmaceutical preparations to some extent and then compounded by mixing and the resulting preparation in an effective dose is administered. A pharmaceutical product which is prepared in one drug by mixing several kinds of ingredients having similar or different efficacies is referred to as "a drug combination." It is still further possible that the cholinesterase inhibitor and the selective serotonin reuptake inhibitor, or milnacipran are made into separate pharmaceutical preparations and then they are provided as a kit. Manufacture of a pharmaceutical preparation is able to be carried out based on the art which is commonly used by persons skilled in the art.

In one embodiment, the pharmaceutical composition for treating depression (e.g., major depression) of the invention is used for the treatment (protocol) comprising administering a selective serotonin reuptake inhibitor, and further administering a cholinesterase inhibitor. In the primary treatment of administering the selective serotonin reuptake inhibitor to a patient prior to administering the cholinesterase inhibitor, any other antidepressant or other drugs can be administered to the patient.

The concrete protocol for sequential administration of the drugs in this embodiment is not limited to, but preferably includes, administering the selective serotonin reuptake inhibitor at least one time in a time range (A) from one to four weeks, (B) from four to six weeks, or (C) from six to ninety weeks from the first administration of the drug after the first diagnosis, and subsequently administering the cholinesterase inhibitor at least one time at interval of several weeks (preferably, (a) one to four weeks, (b) four to six weeks, or (c) six to ninety weeks) from the first administration of the selective serotonin reuptake inhibitor after the first diagnosis.

The term "first diagnosis" refers to the case where the patient first consults a medical institution or clinic, the case where the patient previously consulted a medical institution or clinic and cured or terminated the treatment, then newly consulted a doctor; the case where the patient who is necessary to be treated continuously, stopped the treatment without consultation for more than several months; or the case where the patient consults to the second or third or more medical institution or clinic.

In another embodiment, the pharmaceutical composition for treating depression of the invention is used for the treatment (protocol) comprising administering a drug containing duloxetine, and further administering a cholinesterase inhibitor. In the primary treatment of administering the drug containing milnacipran to a patient prior to administering the cholinesterase inhibitor, any other antidepressant or other drugs can be administered to the patient. In the primary treatment of administering the drug containing milnacipran to the patient, the secondary treatment of further administering the cholinesterase inhibitor can be started. The concrete protocol for sequential administration of the drugs in this embodiment is not limited to, but preferably includes administering a drug containing milnacipran at least one time in a time range (A) from one to four weeks, (B) from four to six weeks, or (C) from six to ninety weeks from the first administration of the drug after the first diagnosis, and subsequently administering the cholinesterase inhibitor at least one time at interval of several weeks (preferably, (a) one to four weeks, (b) four to six weeks, or (c) six to ninety weeks) from the first administration of a drug containing milnacipran after the first diagnosis. The term "first diagnosis" refers to the case where the patient first consults a medical institution or clinic; the case where the patient previously consulted to a medical institution or a clinic and cured or terminated the treatment, then newly consults to a doctor; the case where the patient who is necessary to be treated continuously, stopped the treatment without consultation for more than several months; or the case where the patient consults to the second or third or more medical institution or clinic.

There is no particular limitation for the dosage form of the pharmaceutical composition used for the combination therapy of the invention and oral or parenteral administration is possible. In combination use or in compounding, dosage form or dose of each component for the combination use or for the compounding may be different. Therapeutically effective dose may be determined by degree or history of the disease, age, body weight or sex of the patient and other conditions and ability therefor is naturally included in the common technical knowledge of persons skilled in the art.

A pharmaceutical preparation may be prepared in an appropriate dosage form selected, according to need, from generally-known dosage forms such as a tablet, a coated tablet, a pill, a liquid, a suspension, an emulsion, a granule, a capsule, an injectable solution, a suppository, and a spray. Among them, a dosage form capable of oral administration is preferred.

With regard to a carrier used for the manufacture of such a pharmaceutical preparation, there may be used commonly used ones such as excipient, binder, disintegrating agent, lubricant, coloring agent and corrigent and, if necessary, there may be also used, for example, stabilizer, emulsifier, absorption promoter, surfactant, pH adjusting agent, antiseptic, antioxidant, filler, moisturizer, surface activator, dispersing agent, buffer, preservative, dissolving aid and soothing agent. It is possible that components which are commonly used as materials for pharmaceutical preparations are compounded and made into a pharmaceutical preparation by a conventional method. Examples of usable nontoxic components as such are animal and plant oil such as soybean oil, beef tallow and synthetic glyceride; hydrocarbon such as liquid paraffin, squalane and solid paraffin; ester oil such as octyldodecyl myristate and isopropyl myristate, higher alcohol such as cetostearyl alcohol and behenyl alcohol; silicone resin; silicone oil; surfactant such as polyoxyethylene fatty acid ester, sorbitan fatty acid ester, glycerol fatty acid ester, polyoxyethylene sorbitan fatty acid ester, polyoxyethylene hydrogenated castor oil and polyoxyethylene-polyoxypropylene block copolymer; water-soluble polymer such as polyethylene glycol, polyvinylpyrrolidone and methyl cellulose; lower alcohol such as ethanol and isopropanol; polyhydric alcohol (polyol) such as glycerol, propylene glycol, dipropylene glycol, sorbitol and polyethylene glycol; saccharide such as glucose and sucrose; inorganic powder such as silicic acid anhydride, aluminum magnesium silicate and aluminum silicate; inorganic salt such as sodium chloride and sodium phosphate; and pure water.

Examples of the excipient are lactose, fructose, com starch, sugar, glucose, mannitol, sorbitol, crystalline cellulose and silicon dioxide; examples of the binder are polyvinyl alcohol, polyvinyl ether, methyl cellulose, ethyl cellulose, acacia, tragacanth, gelatin, shellac, hydroxypropyl methyl cellulose, hydroxypropyl cellulose, polyvinylpyrrolidone, polypropylene glycol-polyoxyethylene block polymer and meglumine; examples of the disintegrating agent are starch, agar, gelatin powder, crystalline cellulose, calcium carbonate, sodium hydrogen carbonate, calcium citrate, dextrin, pectin and carboxymethyl cellulose calcium; examples of the lubricant are magnesium stearate, talc, polyethylene glycol, silica and hydrogenated plant oil; examples of the coloring agent are those which are allowed to be added to drugs; and examples of the corrigent are cocoa powder, menthol, aromatic powder, peppermint oil, borneol and cinnamon powder. The above-mentioned component may be a salt or a hydrate thereof.

In the oral preparation, excipient and others, if necessary, such as binder, disintegrating agent, lubricant, coloring agent and corrigent are added to the effective ingredients used in the present invention and the mixture is made by a common method into, for example, diluted powder, fine particles, granules, tablets, coated tablets or capsules In the case of tablets and granules, they may be appropriately subjected to coating such as sugars coating if necessary. In the case of syrup and injection preparation, pH adjusting agent, dissolving agent, isotonizing agent or the like and, if necessary, dissolving aid, stabilizer of the like are added thereto followed by making into a pharmaceutical preparation by a conventional method.

With regard to donepezil (ARICEPT®), its dose range is 0.01 to 0.75 mg/kg/day. With regard to tacrine (COGNEX®), its dose range is 0.1 to 2.3 mg/kg/day. With regard to rivastigmine (EXELON^{™}), its dose range is 0.1 to 0.5 mg/kg/day. With regard to galanthamine (REMINYL®), its dose range is 0.05 to 1.0 mg/kg/day. With regard to metrifonate (ProMem^{™}), its dose rang is 0.1 to 2.0 mg/kg/day. With regard to neostigmine, its dose range is 0.1 to 2.0 mg/kg/day. With regard to physostigmine (SYNAPTON™), its dose range is 0:01 to 0.4 mg/kg/day.

With regard to fluoxetine (PROZAC®), its dose range is 0.01 to 1.4 mg/kg/day and, preferably, 0.15 to 0.7 mg/kg/day. With regard to escitaloprain (LEXAPRO®), its dose range is 0.08 to 0.8 mg/kg/day and, preferably, 0.15 to 0.5 mg/kg/day. With regard to fluvoxamine (LUVOX®), its dose range is 0.3 to 8.5 mg/kg/day and, preferably, 0.8 to 5.0 mg/kg/day. With regard to paroxetine (PAXIL®), its dose range is 0.3 to 0.85 mg/kg/day and, preferably, 0.3 to 0.5 mg/kg/day. With regard to sertraline (ZOLOFT®), its dose range is 0.3 to 8.5 mg/kg/day and, preferably, 0.8 to 3.3 mg/kg/day.

With regard to milnacipran (TOLEDOMIN^{™}), its dose range is 0.15 to 3.0 mg/kg/day and, preferably, 0.8 to 1.7 mg/kg/day.

In accordance with the invention, there is also provided a pharmaceutical product comprising a drug containing a cholinesterase inhibitor; a drug containing a selective serotonin reuptake inhibitor, or milnacipran, and a label, instruction and/or a package insert that indicate the direction for use in a treatment of depression or major depression of both drugs in the combination.

In that case, each of the cholinesterase inhibitor, the selective serotonin reuptake inhibitors, milnacipran may be included in the drug according to the invention as a separate preparation or, preferably, as a separate unit dosage form.

With regard to the directions for the combination use of both drugs, examples thereof are information for use and dosage such as daily dose and frequency, route of administration for each drug. When the pharmaceutical product of the present invention contains only one of the drugs, information concerning another drug which is to be used in combination may be mentioned in the package insert.

### Examples

Examples which will be shown below are mere exemplifications and they just intend to illustrate the present invention in detail together with the above-mentioned preferred embodiments and do not limit the present invention thereto. Any person skilled in the art is able to modify the present invention without departing from the spirit of the present invention and the modification as such is also included within a scope of the present invention.

### Example 1

A patient of the second case (44 years age; male; company employee; married, two children) suffered from slow work of mind, low power of concentration, insomnia; psychomotor agitation and hypobulia after three months from his type of job changed after consolidation and reorganization of his company. He was suggested by his company doctor to have a temporary leave from office, was introduced the inventor's clinic and visited the inventor after one month from appearance of depression. He was diagnosed to be major depression. In the first week: 10 mg of PAXIL^{®} and 5 mg of Benzalin; HAM-D point 22. In the second week and thereafter: 20 mg of PAXIL^{®} and 10 mg of Benzalin. When the fourth week finished, psychomotor agitation and feeling of uneasiness were improved and HAM-D point became 14. From the sixth week, 5 mg of ARICEPT^{®} was added to the above. From the seventh day since administration of ARICEPT^{®}, subjective improvement was noted and, in the eighth week, HAM-D point was improved to 4. When the twelfth week finished, he returned to his office. The above drugs were administered for one month more. When administration of ARICEPT^{®} was ceased thereafter, depression became bad after two weeks. The administration was then restarted and, from the fifth day, improvement began to be noted and, after two weeks, improvement to the former level was noted.

### Example 2

A patient of the fourth case (24 years age; female; post-graduate student; unmarried) suffered from insomnia, hypobulia, body weight decrease, low power of concentration, thought inhibition and depressive mood due to her difficulty in drafting her master's thesis. She visited the inventor after two months and a half since appearance of the symptoms. In the initial diagnosis (medical examination), she met the diagnostic standard for major depression. HAM-D point was 24. From the first to the fourth weeks: 100 mg of ZOLOFT^{®} and 1 mg of RIVOTRIL^{®}. Depression was improved to some extent. HAM-D point was 14. However, she was still unable to reopen her research. From the fifth week, 3 mg of ARICEPT^{®} was added to the above and administered for two weeks whereupon some subjective improvement in depression was noted although it was still unable to reopen the research (experiments). Therefore, ARICEPT^{®} was increased to 5 mg from the seventh week whereupon a subjective improvement in depression was noted within one week and, in the ninth week, she was able to return to the research, HAM-D point was 6. When ARICEPT^{®} was ceased in the thirteenth week, worsening of depression was noted on the fifth day and, when 5 mg of ARICEPT^{®} was reopened from the fourteenth week, she returned to the original level within one week.

### Example 3

A patient of the fifth case (25 years age; male (unmarried); company employee (programmer); no special anamnesis) became a company employee after graduating from a university and worked as a programmer. After one year and a half from being employed, efficiency of work became bad and suffered from low mental capacity, low power of concentration, hypobulia, insomnia, unwillingness to do and thought suppression. Loss of joy and desire for death occurred. No physical abnormality was found. In the initial diagnosis (medical examination), Hamilton's rating scale was 23 points and he was diagnosed to be major depression. In the first stage, therapy was started with 50 mg of milnacipran and 1.5 mg of etizolam. After one week, insomnia was somewhat improved and Hamilton's rating scale was 20 points. On the eighth day and thereafter, dose was raised to 100 mg of milnacipran and 15 mg of etizolam. After more two weeks later (after three weeks from the initiation of the therapy), Hamilton's rating scale was 19 points. After that, milnacipran was increased to 150 mg but, due to the side effect (tendency to urinary retention), milnacipran was decreased to 100 mg in the fourth week. In Hamilton's rating scale for depression, retardation in thought was not improved at all When four weeks elapsed from initiation of the therapy, administration of 5 mg of ARICEPT^{®} was added to 100 mg of milnacipran and 1.5 g of etizolam. After one week from the initiation of administration of ARICEPT^{®} (five weeks after initiation of the therapy), subjective improvement was noted and, after two weeks from initiation of administration of 5 mg of ARICEPT^{®} (after six weeks from initiation of the therapy), Hamilton's rating scale decreased to 7 points. After four weeks from initiation of administration of ARICEPT^{®} (after eight weeks from initiation of the therapy), ARICEPT^{®} was decreased to 3 mg whereupon depression worsened one week thereafter whereby ARICEPT^{®} was returned to 5 mg. After that, recovery was noted and he has been able to work again.

### Example 4

A patient of the seventh case (45 years age; male (married); public official; no particular anamnesis) was changed his job before three months from the first medical examination whereby human relations became bad, efficiency in his work lowered, thinking power lowered, power of concentration lowered, pleasant thing in the past was now unable to be enjoyed, hypobulia was noted, insomnia appeared and desired for-death was also available. In the initial medical examination, Hamilton's rating scale for depression was 22 points and he was diagnosed as depression (major depression). In the first week, 50 mg of milnacipran and 0.125 mg of TRIOZOLAM^{™} were administered. In the second week, 150 mg of milnacipran and 0.125 mg of triozolam were administered. Hamilton's rating scale for depression when the above was continued for five weeks was 18 points. When 5 mg of ARICEPT^{®} was added from the sixth week, subjective improvement was noted on the tenth day and, on the fourteenth day (after seven weeks from initiation of the therapy), psychomotor inhibition was significantly improved (recovered to the normal) and Hamilton's rating scale for depression became 6 points. ARICEPT^{®} was ceased after two months from the initiation of administration of 5 mg of ARICEPT^{®}. Within one week after that, subjective depression became bad and, after two weeks, depression became bad objectively as well. When ARICEPT^{®} was administered again, recovery to the state before cease of ARICEPT^{®} was noted on the twelfth day.

## Claims

1. A combination of donepezil or a pharmaceutically acceptable salt thereof, and
(i) fluoxetine, fluvoxamine, paroxetine, sertraline or escitalopram, or a pharmaceutically acceptable salt thereof; or
(ii) milnacipran, an enantiomer thereof, a diastereomer thereof, a pharmaceutically acceptable salt thereof, an enantiomer of a pharmaceutically acceptable salt thereof, or a diastereomer of a pharmaceutically acceptable salt thereof
for use in the treatment of major depression.

2. The combination for use according to claim 1, wherein (i) or (ii) is administered for a period of time (A) from one to four weeks, (B) from four to six weeks, or (C) from six to ninety weeks from the first administration thereof after the first diagnosis of depression; and subsequently donepezil or a pharmaceutically acceptable salt thereof is administered (a) from one to four weeks, (b) from four to six weeks, or (c) from six to ninety weeks from the first administration of (i) or (ii) after the first diagnosis of depression.

3. The combination for use according to claim 2, wherein (1) the administration of donepezil or a pharmaceutically acceptable salt thereof is discontinued or the dosage of donepezil or a pharmaceutically acceptable salt thereof is reduced for several days to four weeks, and subsequently (2) donepezil or a pharmaceutically acceptable salt thereof is readministered and/or the dosage of donepezil or a pharmaceutically acceptable salt thereof is increased.

4. The combination for use according to any one of claims 1 to 3, wherein donepezil or the pharmaceutically acceptable salt thereof and (i) or (ii) are administered to the patient as (1) separate pharmaceutical compositions; or as (2) a single pharmaceutical composition comprising both donepezil or the pharmaceutically acceptable salt thereof and (i) or (ii).

5. The combination for use according to any one of claims 1 to 4, wherein the pharmaceutically acceptable salt of donepezil is donepezil hydrochloride.

6. The combination for use according to any one of claims 1 to 4, wherein (i) is fluoxetine hydrochloride, fluvoxamine maleate, paroxetine hydrochloride, or sertraline hydrochloride.

7. The combination for use according to any one of claims 1 to 4, wherein (ii) is milnacipran hydrochloride.

8. A pharmaceutical composition comprising donepezil or a pharmaceutically acceptable salt thereof, and
(i) fluoxetine, fluvoxamine, paroxetine, sertraline or escitalopram, or a pharmaceutically acceptable salt thereof; or
(ii) milnacipran, an enantiomer thereof, a diastereomer thereof, a pharmaceutically acceptable salt thereof, an enantiomer of a pharmaceutically acceptable salt thereof, or a diastereomer of a pharmaceutically acceptable salt thereof
for use in the treatment of major depression.

9. A kit comprising a first composition which comprises donepezil or a pharmaceutically acceptable salt thereof and a second composition which comprises
(i) fluoxetine, fluvoxamine, paroxetine, sertraline or escitalopram, or a pharmaceutically acceptable salt thereof; or
(ii) milnacipran, an enantiomer thereof, a diastereomer thereof, a pharmaceutically acceptable salt thereof, an enantiomer of a pharmaceutically acceptable salt thereof, or a diastereomer of a pharmaceutically acceptable salt thereof
for use in the treatment of major depression.

10. The use of donepezil or a pharmaceutically acceptable salt thereof in combination with at least one of (i) fluoxetine, fluvoxamine, paroxetine, sertraline or escitalopram, or a pharmaceutically acceptable salt thereof; or (ii) milnacipran or a pharmaceutically acceptable salt thereof for the preparation of a medicament for the treatment of major depression.

## Patentansprüche

1. Kombination von Donepezil oder eines pharmazeutisch akzeptablen Salzes davon und
(i) Fluoxetin, Fluvoxamin, Paroxetin, Sertralin oder Escitalopram oder eines pharmazeutisch akzeptablen Salzes davon; oder
(ii) Milnacipran, ein Enantiomer davon, ein Diastereomer davon, ein pharmazeutisch akzeptablen Salzes davon, ein Enantiomer eines pharmazeutisch akzeptablen Salzes davon oder ein Diastereomer eines pharmazeutisch akzeptablen Salzes davon
zur Verwendung in der Behandlung von schwerer Depression.

2. Kombination zur Verwendung gemäß Anspruch 1, wobei (i) oder (ii) für folgende Zeitperioden verabreicht wird (A)von einer bis 4 Wochen, (B) von 4 bis 6 Wochen oder (C) von 6 bis 90 Wochen von deren erster Verabreichung nach der ersten Diagnose von Depression; und anschließend Donepezil oder ein pharmazeutisch akzeptables Salz davon wie folgt verabreicht wird (a) von einer bis 4 Wochen, (b) von 4 bis 6 Wochen oder (c) von 6 bis 90 Wochen von der ersten Verabreichung von (i) oder (ii) nach der ersten Diagnose von Depression.

3. Kombination zur Verwendung gemäß Anspruch 2, wobei (1) die Verabreichung von Donepezil oder eines pharmazeutisch akzeptablen Salzes davon unterbrochen wird oder die Dosierung von Donepezil oder eines pharmazeutisch akzeptablen Salzes davon für mehrere Tage bis 4 Wochen reduziert wird und anschließend (2) Donepezil oder ein pharmazeutisch akzeptables Salz davon wieder verabreicht wird oder die Dosis von Donepezil oder eines pharmazeutisch akzeptablen Salzes davon erhöht wird.

4. Kombination zur Verwendung gemäß irgendeinem der Ansprüche 1 bis 3, wobei Donepezil oder das pharmazeutisch akzeptable Salz davon und (i) oder (ii) dem Patienten wie folgt verabreicht wird: (1) als separate pharmazeutische Zusammensetzung oder als (2) als eine einfache pharmazeutische Zusammensetzung, die sowohl Donepezil oder das pharmazeutisch akzeptable Salz davon als auch (i) oder (ii) umfasst.

5. Kombination zur Verwendung gemäß irgendeinem der Ansprüche 1 bis 4, wobei das pharmazeutisch akzeptable Salz von Donepezil Donepezilhydrochlorid ist.

6. Kombination zur Verwendung gemäß irgendeinem der Ansprüche 1 bis 4, wobei (i) Fluoxetinhydrocholorid, Fluovoxaminmaleat, Paroxetinhydrochlorid oder Sertralinhydrochlorid ist.

7. Kombination zur Verwendung gemäß irgendeinem der Ansprüche 1 bis 4, wobei (ii) Milnacipranhydrochlorid ist.

8. Pharmazeutische Zusammensetzung, umfassend Donepezil oder ein pharmazeutisch akzeptables Salz davon und
(i) Fluoxetin, Fluvoxamin, Paroxetin, Sertralin oder Escitalopram oder eines pharmazeutisch akzeptables Salz davon; oder
(ii) Milnacipran, ein Enantiomer davon, ein Diastereomer davon, ein pharmazeutisch akzeptables Salz davon, ein Enantiomer eines pharmazeutisch akzeptablen Salzes davon oder ein Diastereomer eines pharmazeutisch akzeptablen Salzes davon
zur Verwendung in der Behandlung von schwerer Depression.

9. Kit, umfassend eine erste Zusammensetzung, die Donepezil oder ein pharmazeutisch akzeptables Salz davon und eine zweite Zusammensetzung, die
(i) Fluoxetin, Fluvoxamin, Paroxetin, Sertralin oder Escitalopram oder eines pharmazeutisch akzeptables Salz davon; oder
(ii) Milnacipran, ein Enantiomer davon, ein Diastereomer davon, ein pharmazeutisch akzeptables Salz davon, ein Enantiomer eines pharmazeutisch akzeptablen Salzes davon oder ein Diastereomer eines pharmazeutisch akzeptablen Salzes davon umfasst,
zur Verwendung in der Behandlung von schwerer Depression.

10. Verwendung von Donepezil oder eine pharmazeutisch akzeptablen Salzes davon in Kombination mit mindestens einem aus (i) Fluoxetin, Fluvoxamin, Paroxetin, Sertralin oder Escitalopram oder eines pharmazeutisch akzeptablen Salzes davon; oder (ii) Milnacipran oder eines pharmazeutisch akzeptablen Salzes davon zur Herstellung eines Medikaments zur Behandlung von schwerer Depression.

## Revendications

1. Combinaison de donépézil ou d'un sel pharmaceutiquement acceptable de celui-ci ; et
(i) de fluoxétine, fluvoxamine, paroxétine, sertraline ou d'escitalopram, ou d'un sel pharmaceutiquement acceptable de ceux-ci ; ou
(ii) de milnacipran, un énantiomère de celui-ci, un diastéréomère de celui-ci, un sel pharmaceutiquement acceptable de celui-ci, un énantiomère d'un sel pharmaceutiquement acceptable de celui-ci, ou un diastéréomère d'un sel pharmaceutiquement acceptable de celui-ci
pour utilisation dans le traitement d'une dépression majeure.

2. Combinaison pour utilisation selon la revendication 1, dans laquelle (i) ou (ii) est administré(e) pendant une durée (A) allant d'une à quatre semaines, (B) de quatre à six semaines, ou (C) de six à nonante semaines à partir de sa première administration après le premier diagnostic de dépression ; et par la suite le donépézil ou un sel pharmaceutiquement acceptable de celui-ci est administré (a) pendant une à quatre semaines, (b) pendant quatre à six semaines, ou (c) pendant six à nonante semaines à partir de la première administration de (i) ou (ii) après le premier diagnostic de dépression.

3. Combinaison pour utilisation selon la revendication 2, dans laquelle (1) l'administration de donépézil ou d'un sel pharmaceutiquement acceptable de celui-ci est discontinue ou la posologie de donépézil ou d'un sel pharmaceutiquement acceptable de celui-ci est diminuée entre plusieurs jours à quatre semaines, et par la suite (2) le donépézil ou un sel pharmaceutiquement acceptable de celui-ci est réadministré et/ou la posologie de donépézil ou d'un sel pharmaceutiquement acceptable de celui-ci est augmentée.

4. Combinaison pour utilisation selon l'une quelconque des revendications 1 à 3, dans laquelle le donépézil ou le sel pharmaceutiquement acceptable de celui-ci et (i) ou (ii) sont administrés au patient comme (1) compositions pharmaceutiques séparées ; ou comme (2) composition pharmaceutique unique comprenant à la fois le donépézil ou le sel pharmaceutiquement acceptable de celui-ci et (i) ou (ii).

5. Combinaison pour utilisation selon l'une quelconque des revendications 1 à 4, dans laquelle le sel pharmaceutiquement acceptable de donépézil est le chlorhydrate de donépézil.

6. Combinaison pour utilisation selon l'une quelconque des revendications 1 à 4, dans laquelle (i) est le chlorhydrate de fluoxétine, le maléate de fluvoxamine, le chlorhydrate de paroxétine, ou le chlorhydrate de sertraline.

7. Combinaison à utiliser selon l'une quelconque des revendications 1 à 4, dans laquelle (ii) est le chlorhydrate de milnacipran.

8. Composition pharmaceutique comprenant le donépézil ou un sel pharmaceutiquement acceptable de celui-ci, et
(i) de la fluoxétine, fluvoxamine, paroxétine, sertraline ou l'escitalopram, ou un sel pharmaceutiquement acceptable de ceux-ci ; ou
(ii) du milnacipran, un énantiomère de celui-ci, un diastéréomère de celui-ci, un sel pharmaceutiquement acceptable de celui-ci, un énantiomère d'un sel pharmaceutiquement acceptable de celui-ci, ou un diastéréomère d'un sel pharmaceutiquement acceptable de celui-ci
pour utilisation dans le traitement de dépression majeure.

9. Kit comprenant une première composition qui comprend le donépézil et un sel pharmaceutiquement acceptable de celui-ci et une deuxième composition qui comprend
(i) de la fluoxétine, fluvoxamine, paroxétine, sertraline ou de l'escitalopram, ou un sel pharmaceutiquement acceptable de ceux-ci ; ou
(ii) du milnacipran, un énantiomère de celui-ci, un diastéréomère de celui-ci, un sel pharmaceutiquement acceptable de celui-ci, un énantiomère d'un sel pharmaceutiquement acceptable de celui-ci, ou un diastéréomère d'un sel pharmaceutiquement acceptable de celui-ci
pour utilisation dans le traitement de dépression majeure.

10. Utilisation de donépézil ou d'un sel pharmaceutiquement acceptable de celui-ci dans une combinaison avec au moins l'un de (i) la fluoxétine, la fluvoxamine, la paroxétine, la sertraline ou l'escitalopram, ou un sel pharmaceutiquement acceptable de ceux-ci ; ou (ii) le milnacipran ou un sel pharmaceutiquement acceptable de celui-ci pour la préparation d'un médicament pour le traitement de dépression majeure.
